(19) Europäisches Patentamt
European Patent Office
Office européen des brevets

(11) EP 0 593 897 B1

(12) # EUROPÄISCHE PATENTSCHRIFT

(45) Veröffentlichungstag und Bekanntmachung des Hinweises auf die Patenterteilung:
**17.04.1996 Patentblatt 1996/16**

(51) Int. Cl.$^6$: **A61K 31/685**, A61K 47/02, A61K 47/10, A61K 47/12

(21) Anmeldenummer: **93114672.4**

(22) Anmeldetag: **13.09.1993**

(54) **Stabilisierte Alkylphosphocholinlösungen in Glycerinalkylethern**
Stabilized solutions of alkylphosphocholine in glycerolalkylether
Solution stabilisées d'alkylphospholine dans des glycérolalkyléthers

(84) Benannte Vertragsstaaten:
**AT BE CH DE DK ES FR GB GR IE IT LI LU MC NL PT SE**

(30) Priorität: **23.10.1992 DE 4235911**

(43) Veröffentlichungstag der Anmeldung:
**27.04.1994 Patentblatt 1994/17**

(73) Patentinhaber: **ASTA Medica Aktiengesellschaft**
**D-01277 Dresden (DE)**

(72) Erfinder:
- **Engel, Jürgen, Prof. Dr.**
  **D-63755 Alzenau (DE)**
- **Wolf-Heuss, Elisabeth, Dr.**
  **D-74821 Mosbach (DE)**
- **Orth, Helmut, Dr.**
  **D-63457 Hanau (DE)**
- **Wichert, Burkhard, Dr.**
  **D-33615 Bielefeld (DE)**
- **Sauerbier, Diester**
  **D-33824 Werter (DE)**

(56) Entgegenhaltungen:
**US-A- 4 837 023**

- **LIPIDS, Band 26, 1991; C. UNGER et al., Seiten 1412-1417**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 593 897 B1

Printed by Rank Xerox (UK) Business Services
2.11.2/3.4

## Beschreibung

Alkylphosphorsäureverbindungen sind bekannte Substanzen, die Antitumorwirkungen besitzen.

DE-OS 33 43 530.8 offenbart die Antitumorwirkung und die präparative Darstellung der Verbindungen. Als Lösungsmittel für diese Wirkstoffe werden Wasser oder physiologisch verträgliche organische Lösungsmittel wie zum Beispiel Glycerinalkylether eingesetzt.

Auch aus der US-Patentschrift 4,837,023 ist eine Zusammensetzung aus Hexadecylphosphocholin und einem Glycerinalkylether zur Tumorbehandlung bekannt.

Für die topische Applikation hat es sich beispielsweise als günstig herausgestellt, den Wirkstoff zusammen mit wenigstens einem Glycerinalkylether mit 2-12 Kohlenstoffatomen im Alkylrest, der in Form einer Ethergruppe an eine der primären oder sekundären OH-Gruppen des Glycerins gebunden vorliegen kann, einzusetzen. Derartige Glycerinalkylether steigern beziehungsweise verbessern die Wirkung der Alkylphosphorsäureverbindungen. Bevorzugt wurden hierbei Glycerinalkylether mit 3-9 C-Atomen allein oder in Mischungen verwendet. Besonders günstige Wirkungen besitzt daher ein Arzneimittel, welches

a) eine oder mehrere Alkylphosphorsäureverbindungen der allgemeinen Formel I

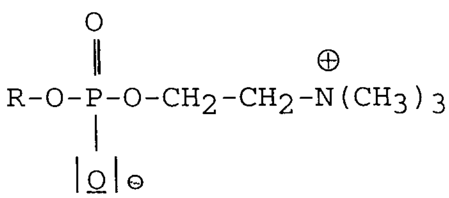

I

worin R die Bedeutungen einer Alkylgruppe mit 12-20 Kohlenstoffatomen hat, die gegebenenfalls eine Doppelbindung oder eine Dreifachbindung enthalten kann,

b) ein Glycerinalkylether der allgemeinen Formel II

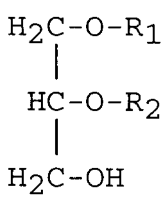

II

in welcher einer der Reste $R_1$ und $R_2$ eine Alkylgruppe mit 2 bis 12 C-Atomen und der andere Rest ein H-Atom bedeutet,

enthält, sowie gegebenenfalls weitere übliche Zusatz- und Verdünnungsmittel.

Vorzugsweise kommt ein Gemisch aus Wasser und einer Glycerinalkylethermischung von Glycerinnonyl- beziehungsweise Glycerinoctylether, Glycerinhexyl- beziehungsweise Glycerinpentylether und Glycerinpropyl- beziehungsweise Glycerinethylether in Frage. Beispielsweise enthält eine entsprechende Formulierung für die topische Anwendung 1-100 mg Alkylphosphorsäureverbindung pro ml Glycerinalkylether beziehungsweise einer entsprechenden Glycerinalkylethermischung mit Wasser. Eine derartige Mischung wird im folgenden auch als Kaskade bezeichnet. Beispielsweise werden Glycerinalkylether-Wasser-Mischungen, welche zum Beispiel Glycerinnonylether, Glycerinhexylether, Glycerinpropylether enthalten, bevorzugt. Vorzugsweise enthalten solche wäßrigen Mischungen 3 der genannten Glycerinalkylether, und zwar einen niederen (Propyl), einen mittleren (Hexyl) und einen höheren (Nonyl), wobei die Gewichtsmenge an dem niederen Ether etwa so groß ist wie die Summe der Gewichtsmengen an den beiden anderen Glycerinalkylethern. Die Wassermenge ist etwa gleich der Menge an dem niederen Glycerinalkylether und beträgt beispielsweise die Hälfte der Gesamtmenge an den vorliegenden Glycerinalkylethern.

Beispiele für solche Glycerinalkylether Wassermischungen sind im folgenden angeführt:

| | Wasser | Glycerinpropylether | Glycerinhexylether | Glycerinnonylether |
|---|---|---|---|---|
| Gewichtsteile | 2 | 2 | 1 | 1 |
| | Wasser | Glycerinethylether | Glycerinpentylether | Glycerinoctylether |
| Gewichtsteile | 2 | 2 | 1 | 1 |

Neben Wasser können noch alle für topische Arzneimittel einsetzbaren Lösungsmittel verwendet werden.

Eine besonders günstige Trägermischung für die Alkylphosphorsäureverbindung Hexadecylphosphocholin besteht aus einer Mischung von etwa 4 Gewichtsteilen Wasser, 4 Gewichtsteilen Glycerinpropylether und je 2 Gewichtsteilen Glycerinhexylether und Glycerinnonylether.

Eine Konservierung derartiger Lösungen ist nicht erforderlich, da in mikrobiologischen Belastungstesten optimale antiseptische Eigenschaften der Lösungen erkannt wurden.
Beispielsweise setzt sich eine 6 %ige Hexadecylphosphocholinlösung wie folgt zusammen:

| | |
|---|---|
| Hexadecylphosphocholin | 0,600 g |
| Glycerin-1-n-propylether | 3,145 g |
| Glycerin-1-n-hexylether | 1,570 g |
| Glycerin-1-n-nonylether | 1,570 g |
| Wasser | 3,145 g |
| | 10,030 g = 10 ml |

Leider stellte sich während der Stabilitätslagerung heraus, daß durch oxidative Prozesse in der Lösung Peroxide entstehen, die später durch weitere Zersetzung zu Säuren und damit pH-Abfall führen. In der Spezifikation wurde der pH-Wert auf 4-6 festgelegt.

pH und Peroxidzahl einer 6 %igen Hexadecylphosphocholinlösung in Kaskade (unbehandelt)

| Ausgangsuntersuchung | -4°C | +2°C | RT | 31°C | 41°C |
|---|---|---|---|---|---|
| pH-Wert | | | 5,6 | | |
| Peroxidzahl | | | 0,4 | | |
| 3 Monate | | | | | |
| pH-Wert | 5,0 | 5,0 | 4,0 | 3,6 | 3,3 |
| Peroxidzahl | 0,81 | 0,95 | 0,91 | 3,6 | 6,0 |
| 6 Monate | | | | | |
| pH-Wert | 7,4 | 6,0 | 3,8 | 3,5 | 3,1 |
| Peroxidzahl | 0,55 | 0,18 | 1,9 | 4,9 | 7,8 |

Zur Eindämmung der Peroxidbildung wurden Hexadecylphosphocholin Kaskade-Lösungen mit Stickstoff begast und nach Abfüllung in 10 ml-Flaschen mit Stickstoff überschichtet. Außerdem wurde versucht, durch Zusatz von Antioxidantien die Peroxidbildung zu verhindern. pH und Peroxidzahl einer 6 %igen Hexadecylphosphocholinlösung in Kaskade (nach Begasung mit Stickstoff).

| Ausgangsuntersuchung | -4°C | +2°C | RT | 31°C | 41°C |
|---|---|---|---|---|---|
| pH-Wert<br>Peroxidzahl | | | 4,6<br>0,0 | | |
| 3 Monate | | | | | |
| pH-Wert<br>Peroxidzahl | 4,6<br>0,1 | 5,0<br>0,11 | 4,2<br>0,97 | 3,9<br>0,94 | 3,6<br>5,1 |
| 6 Monate | | | | | |
| pH-Wert<br>Peroxidzahl | 4,5<br>0,0 | 4,4<br>0,0 | 4,1<br>1,73 | 3,9<br>1,1 | 3,5<br>4,6 |
| RT = Raumtemperatur | | | | | |

Wie ersichtlich, fällt auch hier der pH-Wert ab und der Peroxidgehalt steigt an.
Der Zusatz von Antioxidantien brachte auch keine Verbesserung, wie die folgende Tabelle zeigt.
pH-Wert von Hexadecylphosphocholin Kaskade-Lösungen mit Zusatz von Antioxidantien.

Mit 0,1 % Natriumdisulfit

| | -4°C | +2°C | RT | 31°C | 41°C |
|---|---|---|---|---|---|
| Ausgangs-pH-Wert<br>3 Monate pH-Wert | 3,8 | 3,7 | 3,3 | 3,1 | 2,8 |

Mit 0,01 % Ascorbylpalmitat und 0,05 % $\alpha$-Tocopherol

| | -4°C | +2°C | RT | 31°C | 41°C |
|---|---|---|---|---|---|
| Ausgangs-pH-Wert | | | 3,8 | | |
| 3 Monate pH-Wert | 3,8 | 3,7 | 3,3 | 3,1 | 2,8 |

Es ist durch die Versuche belegt, daß die übliche Formulierung nicht lagerstabil ist.
Auch die Anwendung üblicher Mittel zur Unterdrückung der Peroxidbildung, wie zum Beispiel Begasung mit Stickstoff, um den Sauerstoff aus der Lösung zu entfernen, und der Zusatz von Antioxidantien führte nicht zu lagerstabilen Lösungen.

In einem Versuch wurde zusätzlich zum Antioxidans Natriumdisulfit ein Citratpuffersystem eingesetzt. Nun konnte man feststellen, daß der pH-Wert innerhalb der Spezifikation gehalten werden konnte.

pH-Wert von Miltefosin-Kaskadelösungen mit Zusatz von 0,1 % Natriumdisulfit und Citratpuffer (0,1 molar in der Wasserphase). (Miltefosin ist chemisch 0-Hexadecyl-0-(2-trimethylaminoethyl)phosphat.)

| | -4°C | +2°C | RT | 31°C | 41°C |
|---|---|---|---|---|---|
| Ausgangs-pH-Wert | | | 5,6 | | |
| 3 Monate pH-Wert | 5,6 | 5,6 | 5,3 | 5,1 | 5,2 |

Da Natriumdisulfid als Antioxidans keinen positiven Einfluß ausübte, wurde eine Miltefosin-Kaskadelösung hergestellt, die nur Citratpuffer zur Verminderung der Peroxidbildung enthielt.

Überraschenderweise konnte festgestellt werden, daß durch den Pufferzusatz die Peroxidbildung und damit ein Absinken des pH-Wertes unterdrückt werden konnte.

Hexadecylphosphocholin Kaskade-Lösung mit Citratpuffer

| | -4°C | +2°C | RT | 31°C | 41°C |
|---|---|---|---|---|---|
| Ausgangs-pH-Wert | | | 5,8 | | |
| Peroxidzahl | | | 0,58 | | |
| 3 Monate | | | | | |
| pH-Wert | 5,8 | 5,8 | 5,8 | 5,8 | 5,7 |
| Peroxidzahl | 0,33 | 0,29 | 0,41 | 0,51 | 0,58 |
| 6 Monate | | | | | |
| pH-Wert | 5,8 | 5,8 | 5,8 | 5,7 | 5,6 |
| Peroxidzahl | 0,02 | 0,39 | 0,11 | 0,36 | 0,54 |
| 12 Monate | | | | | |
| pH-Wert | 5,8 | 5,8 | 5,7 | 5,6 | 5,3 |
| Peroxidzahl | 0,06 | 0,04 | 0,18 | 0,11 | 0,3 |

10 ml einer 6 %igen gepufferten Hexadecylphosphochlin Kaskade-Lösung setzen sich wie folgt zusammen:

| | |
|---|---|
| Hexadecylphosphocholin | 0,6000 g |
| D,L-Glycerin-1-n-propylether | 3,1600 g |
| D,L-Glycerin-1-n-hexylether | 1,5800 g |
| D,L-Glycerin-1-n-nonylether | 1,5800 g |
| Citronensäure wasserfrei | 0,0484 g |
| Natriumhydroxid | 0,0227 g |
| Gereinigtes Wasser | 3,0889 g |
| | 10,0800 g = 10 ml |

Weitere geeignete Puffergemische stellen folgende Mischungen dar: Mischungen aus Dinatriumhydrogenphosphat/Citronensäure, Bernsteinsäure/Natriumhydroxid, Kaliumdihydrogenphosphat/Dinatriumhydrogenphosphat, Natriumhydrogenmaleat/Natriumhydroxid, Tris-Maleat/Natriumhydroxid, Kaliumdihydrogenphosphat/Natriumhydroxid. Besonders bevorzugt ist eine Mischung aus Citronensäure und Natriumhydroxid vom pH-Wert 5,3.

Beispiel 1

Gepufferte 6 %ige (G/V) Hexadecylphosphocholin-Lösung für die topische Anwendung

Hexadecylphosphocholin-Lösung wird durch Auflösen von Hexadecylphosphocholin in einem gepufferten, als Kaskade bezeichneten Lösungsmittel, hergestellt.
6,27 kg DL-Glycerin-1-n-ethylether, 3,135 kg DL-Glycerin-1-n-pentylether und 3,135 kg DL-Glycerin-1-n-octylether werden gemischt und in dieser Mischung 1,19 kg Hexadecylphosphocholin gelöst.

Herstellung von Citratpuffer pH 5,3
0,0965 kg Citronensäure wasserfrei werden in 5,8 kg gereinigtem Wasser und 0,047 kg Natriumhydroxid in 0,3 kg gereinigtem Wasser gelöst. Die Natriumhydroxidlösung wird nun der Citronensäurelösung bis zum Einreichen eines pH-Wertes von 5,3 zugesetzt. Anschließend wird mit Wasser auf 6,27 kg aufgefüllt. 6,27 kg Citratpuffer werden nun mit 13,73 kg Lösung des Hexadecylphosphocholins in dem Ethergemisch vereinigt. Unter Stickstoffbegasung wird eine

einheitliche Lösung hergestellt. Die Lösung wird über ein Membranfilter, Porenweite 0,2 µm filtriert, zu 10 ml in braune Tropfflaschen abgefüllt und mit Pipette und Schutzkappe verschlossen.

## Patentansprüche

1. Stabilisierte Lösungen von Alkylphosphocholinen und Glycerinalkylethern in Wasser, bestehend aus ein oder mehreren Alkylphosphocholinen der Formel

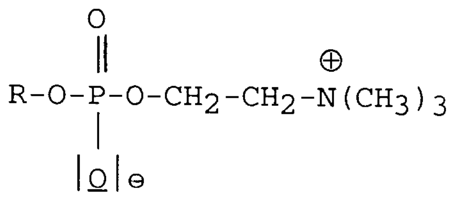

I

worin R eine Alkylgruppe mit 12-20 C-Atomen bedeutet, und Glycerinalkylethern der Formel

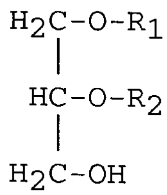

II

worin einer der Reste $R_1$, $R_2$ eine Alkylgruppe mit 2-12 C-Atomen und der andere Rest ein H-Atom bedeutet, gekennzeichnet durch eine zugesetzte Pufferlösung.

2. Lösung gemäß Anspruch 1,
dadurch gekennzeichnet, daß
die Pufferlösung aus einer Lösung von Citronensäure und Natriumhydroxid in Wasser besteht.

3. Lösung gemäß den Ansprüchen 1 und 2,
dadurch gekennzeichnet, daß
der pH-Wert der Lösung zwischen 4 und 6 liegt.

4. Verfahren zur Herstellung einer Lösung gemäß den Ansprüchen 1 bis 3,
dadurch gekennzeichnet, daß
man ein oder mehrere Alkylphosphocholine gemäß Formel I in einer Mischung aus Glycerinalkylethern gemäß Formel II löst und eine Pufferlösung zusetzt, so daß ein pH-Wert von 4 bis 6 erreicht wird.

5. Verfahren gemäß Anspruch 4,
dadurch gekennzeichnet, daß
als Pufferlösung eine Lösung von Citronensäure und Natriumhydroxid verwendet wird.

6. Arzneimittel zur Tumortherapie,
dadurch gekennzeichnet, daß
es eine gepufferte Lösung gemäß Anspruch 1 darstellt.

7. Verwendung der Lösung gemäß Anspruch 1 zur Herstellung eines topischen Mittels zur Bekämpfung von Tumorerkrankungen.

## Claims

1. Stabilised solutions of alkylphosphocholines and glycerol alkyl ethers in water consisting of one or more alkylphosphocholines of the formula

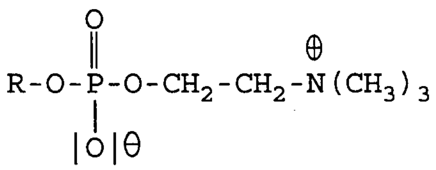

I

in which R means an alkyl group with 12 to 20 C atoms, and glycerol alkyl ethers of the formula

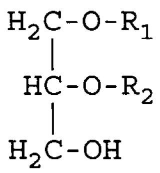

II

in which one of the residues $R_1$, $R_2$ means an alkyl group with 2 to 12 C atoms and the other residue means an H atom, characterised by an added buffer solution.

2. Solution according to claim 1,
characterised in that
the buffer solution consists of a solution of citric acid and sodium hydroxide in water.

3. Solution according to claims 1 and 2,
characterised in that
the pH value of the solution is between 4 and 6.

4. Process for the production of a solution according to claims 1 to 3,
characterised in that
one or more alkylphosphocholines according to the formula I is/are dissolved in a mixture of glycerol alkyl ethers according to the formula II and a buffer solution is added such that a pH value of 4 to 6 is achieved.

5. Process according to claim 4,
characterised in that
a solution of citric acid and sodium hydroxide is used as the buffer solution.

6. Pharmaceutical preparation for tumour therapy,
characterised in that
it constitutes a buffered solution according to claim 1.

7. Use of the solution according to claim 1 for the production of a topical agent to combat tumorous conditions.

## Revendications

1. Solutions stabilisées d'alcoylphosphocholine et d'éthers d'alcoylglycérol dans l'eau formées d'une ou plusieurs alcoylphosphocholines de formule :

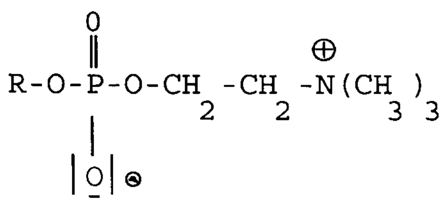

I

dans laquelle R a les significations d'un groupe alcoyle ayant de 12 à 20 atomes de carbone, et d'éthers d'alcoylglycérol de formule II,

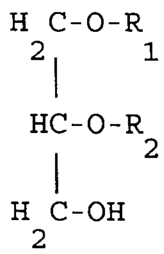

II

dans laquelle un des radiaux $R_1$ et $R_2$ signifie un groupe alcoyle ayant de 2 à 12 atomes de carbone et l'autre radicalsignifie un atome d'hydrogène,
caractérisé par une solution tampon ajoutée.

2. Solution conformément à la revendication 1, caractérisée en ce que la solution tampon est formée d'une solution d'acide citrique et d'hydroxyde de sodium dans l'eau.

3. Solution conformément aux revendications 1 et 2, caractérisée en ce que la valeur du pH de la solution se situe entre 4 et 6.

4. Procédé d'obtention d'une solution conformément aux revendications 1 à 3, caractérisé en ce que l'on dissout une ou plusieurs alcoylphosphocholines conformément à la formule I dans un mélange d'éthers d'alcoylglycérol conformément à la formule II et qu'on ajoute une solution tampon, de sorte qu'une valeur de pH allant de 4 à 6 est atteinte.

5. Procédé conformément à la revendication 4, caractérisé en ce que l'on utilise comme solution tampon une solution d'acide citrique et d'hydroxyde de sodium.

6. Médicament pour la thérapeutique tumorale, caractérisé en ce qu'il représente une solution tamponnée conformément à la revendication 1.

7. Utilisation de la solution conformément à la revendication 1 en vue de l'obtention d'un agent topique pour la lutte contre les maladies tumorales.